# EUROPEAN PATENT SPECIFICATION

(11) **EP 1 690 549 B1**
(45) Date of publication and mention of the grant of the patent: **15.04.2009**
(21) Application number: 04791401.5
(22) Date of filing: 29.10.2004
(51) Int. Cl.: A61K 39/04, A61P 37/04

(54) **IMMUNOTHERAPIC AGENT WHICH IS USED FOR THE COMBINED TREATMENT OF TUBERCULOSIS TOGETHER WITH OTHER PHARMACEUTICALS**
IMMUNTHERAPEUTIKUM FÜR DIE KOMBINIERTE BEHANDLUNG VON TUBERKULOSE MIT ANDEREN PHARMAZEUTIKA
AGENT IMMUNOTHERAPEUTIQUE UTILE DANS LE TRAITEMENT COMBINE DE LA TUBERCULOSE ASSOCIE A D'AUTRES MEDICAMENTS

(30) Priority: 31.10.2003 ES 200302551
(43) Date of publication of application: 16.08.2006
(73) Proprietor: Archivel Farma, SL, 08304 Mataro (Barcelona) (ES)
(72) Inventor: CARDONA IGLESIAS, Pere Joan, P.I. Mata-Rocafonda, 08304 Mataro (ES); AMAT RIERA, Isabel, P.I. Mata-Rocafonda, 08304 Mataro (ES)
(74) Representative: Gislon, Gabriele
(86) International application number: PCT/ES2004/000482
(87) International publication number: WO 2005/042013

(56) References cited:
- WO-A2-00/21983
- RU-C1- 2 153 354
- US-A1- 2002 127 700
- SINHA S. ET AL.: 'Proteome analysis of the plasma membrane of Mycobacterium tuberculosis' COMPARATIVE AND FUNCTIONAL GENOMICS vol. 3, no. 6, 2002, pages 470 - 483, XP002998683
- BHAIRI SM: "DETERGENTS: A guide to the properties and uses of detergents in biological systems" 2001, CALBIOCHEM-NOVABIOCHEM CORPORATION

## Description

### Technical field

This invention refers to a method for preparing an immunotherapeutic agent, which is used for the combined treatment of tuberculosis in association with other drugs. It refers also to the immunotherapeutic agent obtained with the aforementioned method, which is based on cell wall fragments of a virulent *Mycobacterium tuberculosis*-complex strain.

### Background of the invention

Tuberculosis is a chronic infectious disease caused by *Mycobacterium tuberculosis*-complex (MTB-C) bacilli, which currently includes the following species:
*M.tuberculosis, M.bovis, M.microti* and *M.africanum.*

According to the World Health Organization (WHO), there are 8,000,000 new cases of tuberculosis and about 3,000,000 people die every year. It is believed that there are 2,000,000,000 people infected worldwide.

The current vaccine used as a preventive treatment against tuberculosis is based on bacteria from the so-called BCG strain (Calmette-Guerin bacillus), a variety of *M.bovis.*

According to WO-A-03018053, this is the best vaccine currently available to induce immunoprotection against tuberculosis. However, the safety and the effectiveness of this vaccine in humans remain controversial in some countries because it does not completely protect adults against pulmonary tuberculosis.

On the other hand, WO-A-03004520 describes as a known fact that the most effective treatment to fight tuberculosis in infected people, both for those who have and those who have not developed the disease, consists of the administration of several drugs, including isoniazid, for a period of several months.

This prolonged treatment might induce the development of microorganisms resistant to these drugs when the treatment were not completed and, moreover, the aforementioned drugs only act when the bacillus has an active metabolism (i.e., when it is growing) but not when it has a non-active metabolism. This is a significant inconvenient because during tuberculosis infection bacilli coexist in both an active and a non-active metabolism phase.

One possibility to solve these problems, as described in the patent US4724144, consists in the use of an immunotherapeutic agent based on dead *M. vaccae* cells as an adjuvant for the treatment to tuberculosis together with the administration of other drugs, such as rifampicin and isoniazid.

However, patent US6001361 states that such an adjuvant agent has not been used in large-scale vaccination of people against tuberculosis, and there is little information on its effectiveness.

PCT Patent application WO-A-00/21983 discloses extracts containing polypeptides from the cytosol as well as cell wall and cell membrane components of *M. tuberculosis,* which are free of insoluble cell wall.

Russian patent RU-C1-2153354 discloses a vaccine against a tuberculosis, which contains a mixture of TRITON^{®} extract of mycobacterium cell walls from the bacilli Calmette-Guérin and non-specific immunomodulating agent polyoxydonium.

Sinha et al., Comp. Funct. Genom., 2002, 3, 470-483 discloses cell membrane fractions of a virulent *M. tuberculosis* strain, but not to cell wall fragments.

US patent application US-A-2002/127700 discloses a pharmaceutical agent for the treatment of an infection caused by a mycobacterium species, which comprises a cell extract of MTB-C, in particular supernatant fractions, but not insoluble cell wall fractions.

Therefore, an immunotherapeutic agent is needed for the treatment of tuberculosis to act as a coadjuvant for these drugs, and this agent should not induce the development of resistant microorganisms and also generate immunologic response even against bacilli in a non-active phase.

The authors of this invention have discovered a method that allows the preparation of a new immunotherapeutic agent useful for the combined treatment of tuberculosis in association with other drugs. This immunotherapeutic agent contains cell wall fragments of a virulent MTB-C strain that may increase the effectiveness of the associated drugs to generate an effective immunologic response against bacilli that are not in an active metabolism, thus also reducing the risk of resistance.

### Summary of the invention

The object of this invention is to provide a method to obtain an immunotherapeutic agent containing cell wall fragments of a virulent MTB-C strain, useful for the combined treatment of tuberculosis in association with other drugs.

The immunotherapeutic agent obtained using the previous method and the use of this agent for preparing a drug for the combined treatment of tuberculosis in association with other drugs are also included in the object of this invention.

Moreover, another additional object consists of the pharmaceutical compositions made with this immunotherapeutic agent.

### Detailed description of the invention

The authors of this invention have discovered a method to obtain an immunotherapeutic agent that contains cell wall fragments from a virulent *Mycobacterium tuberculosis*-complex (MTB-C) strain comprising the following steps:
- culture of the virulent MTB-C strain for a period of at least three weeks, and
- homogenization of the cell culture in the presence of a non-ionic surfactant selected from the group consisting of alkylphenol ethoxylates and ethoxylated sorbitan esters.

The virulent strain may be any virulent strain of MTB-C, since the tuberculosis bacillus is very stable and no mutations have been described in immunogenic compounds. One of the strains most frequently used by the researchers in this field, and considered as the strain of reference, is the so-called H37Rv strain that for example may be freely obtained from the National Collection of Type Cultures (NCTC), London, Great Britain (deposit number NC007416).

The virulent strain may be cultured by inoculation in culture media well known by the expert in the field. It may be a solid media, such as Middlebrook 7H10 or 7H11-type agar, or a liquid media, such as the Sauton or the Proskauer-Beck culture media.

As regards this invention, the culture must be done for a period of time of at least three weeks, preferably it ranges from 3 to 4 weeks. The temperature of the culture is preferably maintained at between 34° C and 38° C.

Following the completion of the culture, if it has been conducted in a solid phase, the plates are scrapped to obtain the colonies while avoiding media extraction (agar). Nevertheless, if the culture has been conducted in a liquid phase, the cells are concentrated and washed using conventional techniques known by the experts in this field (e.g., centrifugation).

Homogenization of the strains is carried out in a buffered medium at a neutral pH. In this invention, it is important that the homogenization is conducted in the presence of a non-ionic surfactant that favors the obtaining of finely divided cell wall particles and at least partly emulsifies undesired lipidic fractions.

By means of this homogenization process, MTB-C cells break and small fragments of cell wall are obtained.

Homogenization may be carried out using sonication by ultrasounds, or small beads having a diameter of approximately 1 mm (e.g., of silica or silica-zirconium) together with a mechanic homogenizer. A mechanic homogenizer may be used, such as the BEADBEATER model of the company Biospec.

The buffered media is made up, for example, with PBS buffer (saline solution of phosphate buffer).

The non-ionic surfactant used is selected from the group consisting of alkylphenol ethoxylates and ethoxylated sorbitan esters. More preferably non-ionic surfactant is selected from octylphenol ethoxylates. Most preferably, it is used octylphenol ethoxylate with 7-8 mol of ethylene oxide; these may be found in the market under the name TRITON X-114, for example. The concentration of the non-ionic surfactant during homogenization is comprised between 1 and 5% of the total weight homogenized.

The homogenized mass containing the desired cell wall fragments undergoes a conventional treatment in order to separate these fragments both from non-fragmented cells and solubilized compounds.

For example, after separating the silica or silica-zirconium (if used) by decantation, the homogenized product is gently centrifuged at a speed slower than 5,000 rpm in order to remove the non-fragmented cells as sediments.

Then, the resulting supernatant is centrifuged at a higher speed (for example, higher than 15,000 rpm) to remove the solubilized elements that concentrate in the supernatant liquid, whereas the cell wall fragments concentrate in the sediment. Using conventional techniques known by the skill person, such as washing in PBS buffer and centrifugation, this procedure may be repeated several times until a completely clear supernatant is obtained; this is then rejected.

The sediment obtained that contains cell wall fragments is dispersed in PBS buffer and undergoes a chemical (e.g., treatment with formol) or physical (e.g., treatment in autoclave or pasteurization) process to guarantee the total inactivation of the MTB-C cells that could have remained viable after fragmentation and purification.

Finally, the dispersion of cell wall fragments in PBS buffer is distributed into vials and is freeze-dried at a temperature of between -15° C and -25° C and a vacuum of between 0.1 and 0.5 mbar.

Vials with cell wall fragments of MTB-C are thus obtained that form the immunotherapeutic agent of this invention, and are kept at -70° C.

It is also an object of the invention pharmaceutical compositions, which may be prepared using the immunotherapeutic agent of the invention. These may be formulated as an oil-in-water emulsion type (O/W) or in liposome form. Liposome-type pharmaceutical compositions are preferred.

The formation of liposomes may be carried out using conventional techniques, well known by the skill person. For example, liposomes may be obtained mixing the freeze-dried cell wall fragm ents an d the auxiliary 1 lipids in an aqueous medium to form liposomes, and homogenizing the mixture using a standard procedure, such as a high speed shaker.

The auxiliary lipids to form liposomes are widely known by the skill person. In general, they include phospholipids with a net neutral and/or negative charge and sterols.

The phospholipids used may be, for example: phosphatidylcholine, phosphatidylserine and phosphatidylinositol.

Normally the major component in liposomes is phosphatidylcholine, which may be synthesized or isolated from natural sources. A frequently used marketed product is soybean lecithin.

The sterols used in the preparation of liposomes may be, among others, cholesterol and bile salts.

Preferably, liposomes are formed using a mixture of soybean lecithin and sodium cholate.

Optionally, liposomes may contain additives to improve their stability, such as vitamin E, which acts as a lipid antioxidant.

The liposomes obtained show a particle size distribution wherein 99.9 % are smaller than 1 micron.

Liposomes may undergo freeze-drying to obtain the immunotherapeutic agent of the invention as lyophilized liposomes.

The object of the invention also includes the use of the immunotherapeutic agent to prepare a medicament for the combined treatment of tuberculosis in association with other drugs.

Preferably, but not excluding other routes of administration, the immunotherapeutic agent of the invention is administered by parenteral route.

Among the known antituberculosis drugs, the ones that are preferred for the combined treatment with the therapeutic agent of this invention are isoniazid and rifampicin.

The association between the immunotherapeutic agent of the invention and the antituberculosis drugs for the combined treatment of this disease may be carried out simultaneously or sequentially, e.g., by the simultaneous administration of the drugs and the immunotherapeutic agent, or by the previous administration of drugs followed by the administration of the immunotherapeutic agent.

Surprisingly, it has been found that the combined treatment of tuberculosis by the administration of the immunotherapeutic agent of the invention associated with drugs for the treatment of tuberculosis increases the efficacy of these drugs because it generates an immunologic response against bacilli than are not in an active metabolic phase, thus also reducing the risk of developing resistances.

The following examples provide the skill person with a detailed description of specific procedures within the invention.

### Example 1.- Obtaining the immunotherapeutic agent

80 - 100 plates of Middlebrook 7H11-type agar are inoculated with H37Rv culture provided by the National Collection of Type Cultures (NCTC), London, Great Britain (deposit number NC007416). The concentration of the colony- forming units inoculated in each plate is 10⁵-10⁶ UFC. The plates are incubated for 21 days (3 weeks) at a temperature of between 34° C and 38° C.

After incubation, the colonies are removed from the agar plates using a spatula, and being careful in order not to remove the culture medium. Between 15 and 18 g of crude extract are obtained.

The crude extract is dispersed in approximately 20 mL of PBS buffer that contains 4% weight of TRITON X-114. 35 mL of silica-zirconium beads with 1 mm of diameter are added, and then mechanic homogenization is carried out using a BEADBEATER homogenizer manufactured by Biospec.

The homogenization procedure is continued until less than 5 whole bacilli were detected after observation of 100 fields at a magnification of 1000 following staining with the Ziehl-Neelsen technique.

The product resulting from the homogenization is separated from the silica-zirconium beads by decantation. These are washed in a PBS-buffered solution with 4% weight of TRITON X-114, and the liquid from the different washes are collected with the product, thus obtaining a total volume of about 80-100 mL.

Then the product resulting from the homogenization plus the liquid from washing is centrifuged at 3,000 rpm for 30 minutes in a refrigerated centrifuge at 4° C, so as to remove non-fragmented cells from the sediment.

The supernatant is kept.

Then, the supernatant is centrifuged at 15,100 rpm (equivalent to 27,000 g) for 60 minutes at 4° C, thus obtaining a whitish sediment that contains the cell wall fragments.

The sediment is kept, and the yellowish supernatant is removed.

The sediment is first washed in PBS buffer (3 x 3 mL) and then redispersed in 3 mL of the buffer solution, brought to 20 mL with PBS buffer and then centrifuged again at 15,100 rpm for 60 minutes at 4° C.

The supernatant obtained is discarded.

Washing and centrifugation are repeated, and the supernatant obtained is completely clear and is discarded.

The sediment containing the cell wall fragments is washed in PBS buffer (3x3 mL) and redispersed in 12 mL of PBS buffer.

After the centrifugation and the wash, all the volume resulting from the dispersion of cell wall fragments in PBS buffer is collected in a container and is pasteurized by treatment at 65°C for 1 hour.

Then, the mixture is rapidly cooled down in an ice bath, and the cell wall fragment dispersion is distributed into cryotubes at a rate of 1 mL per cryotube.

The cryotubes with the dispersed cell wall fragments are frozen at -70° C and freeze-dried at a temperature of between -15° C and -22° C and a vacuum between 0.180 and 0.400 mbar.

Between 1 and 1.5 g of immunotherapeutic agent are obtained.

### Example 2.- Obtaining liposomes of the immunotherapeutic agent

Between 740 and 770 mg of the product obtained in Example 1 are weighed in a beaker. Then, 20 mL of a dispersion of pharmaceutical quality soybean lecithin in ethanol is added (1 kg of lecithin in 1 liter of absolute ethanol), and 7 mL of a solution of pharmaceutical quality sodium cholate in water (200 g of sodium cholate in 1 liter of bidistilled water).

pH is adjusted to a value between 7.7 and 8 with a solution of HCl 0.997 N.

Liposomes are prepared by homogenization using a high-speed shaker.

The liposome dispersion obtained this way is diluted to 10% in bidistilled water; the pH is adjusted to a value of between 7.1 and 7.3 with a solution of HCl 0.0997 N.

The liposome dispersion is distributed into cryotubes (0.5 mL per tube) and is frozen at -80° C.

Next, they are freeze-dried and it is obtained the immunotherapeutic agent, the object of the invention, in form of liposomes.

### Example 3.- Effectiveness of the immunotherapeutic agent as adjuvant in the treatment with drugs

In the infection model, female BALB/c, 129/Sv, C57BL/6 and DBA/2 mice aged between 6 to 8 weeks old and free from specific pathogen were used.

A virulent strain of *Mycobacterium tuberculosis* was cultured in Proskauer-Beck medium until a middle logarithmic phase was reached and was kept in 1-mL aliquots at -70° C until used.

The mice were inoculated in a Middelbrook aerosol inoculation apparatus that provides approximate inoculums of 10-50 viable bacilli in the lungs.

The bacillary concentration, i.e., the number of viable bacilli, is determined by the incubation of seriated dilutions of homogenized left lung and spleen in Middelbrook 7H11-type agar. Left lung and spleen samples were homogenized in the presence of 1 mL of PBS buffer.

### I) Treatment with one dose of immunotherapeutic agent in liposome form simultaneously with isoniazid

Infected BALB/c type mice were divided into three groups:
- Treated only with isoniazid at a dose of 25 mg /kg per day for 5 days per week for 6 weeks (Control), or
- Treated with isoniazid at a dose of 25 mg /kg per day for 5 days per week for 6 weeks plus an intranasal dose of 180 µg of immunotherapeutic agent in form of liposomes, the object of the invention, or
- Treated with isoniazid at a dose of 25 mg /kg per day for 5 days a week for 6 weeks plus an intraperitoneal 180 µg dose of immunotherapeutic agent in form of liposomes, the object of the invention.

Treatment with the antibiotic isoniazid was started at week 9 and was continued until week 15.

The dose of the immunotherapeutic agent in form of liposomes, the object of the invention, was administered at week 13.

At week 15, the animals were killed and the bacillary concentration in the left lung and the spleen was determined.

The bacillary concentrations, established following the method described before, were significantly lower in the lungs of the vaccinated animals, whereas the results in the spleen were not statistically different compared with the Control group.

The results, expressed as UFC/mL, are shown in Table 1:

**Table 1**

| Mice group | Lung | Spleen |
|---|---|---|
| Control | 7.5±2.89 | 0.75±0.33 |
| Intranasal | ≤ 2±0* | 0.4±0.2 |
| Intraperitoneal | ≤ 2±0* | 0.52±0.44 |
| * = statistically significant value compared to the Control group, p<0.05 | | |

It may be seen that animals treated with the immunotherapeutic agent in form of liposomes, the object of the invention, administered intranasally and intraperitoneally simultaneously with isoniazid showed a considerably lower number of bacilli in the lungs than mice treated with the antibiotic isoniazid alone.

Taking into account that the number of established bacilli includes all bacilli, both those that are in an active phase and those that are in a non-active phase, the treatment with the immunotherapeutic agent in liposome form would allow to reduce the time of treatment with the antibiotic, since it considerably reduces the number of bacilli that may change with time into an active phase.

### II) Treatment with three doses of liposomed immunotherapeutic agent following treatment with rifampicin and isoniazid

Infected 129/Sv-type mice were divided into two groups:
- Treated with isoniazid at a dose of 25 mg/kg per day for 5 days per week for four weeks and rifampicin at a dose of 10 mg/kg per day for 5 days per week for four weeks (Control), and
- Also treated with three 180 µg doses of immunotherapeutic agent in liposome form, the object of the invention, administered subcutaneously following treatment with rifampicin and isoniazid

The treatment with the antibiotic isoniazid was begun at week 9 and was continued for 4 weeks. At week 13, the treatment with rifampicin was started and concluded at week 17.

At weeks 17, 19 and 21, three doses of the immunotherapeutic agent in liposome form, the object the invention, were administered.

At week 22, the animals were killed and the bacillary concentrations in the left lung and in the spleen were established.

The bacillary concentration was significantly lower in the lungs of vaccinated animals compared with the Control group, whereas no significant differences were found in the spleen between the Control group mice and those who were also treated with the immunotherapeutic agent in liposome form.

The results, expressed in log₁₀ UFC/mL, are shown in Table 2:

**Table 2**

| Mice group | Lung | Spleen |
|---|---|---|
| Control | 2.67±0.83 | 2.35±1.18 |
| Subcutaneous | 1.61±0.58* | 1.37±1.02 |
| * = statistically significant value compared to the Control group, p<0.05 | | |

It may be seen that the mice treated with the immunotherapeutic agent in liposome form, the object of the invention, administered subcutaneously and after treatment with the antibiotics isoniazid and rifampicin showed a considerably lower number of bacilli in the lungs than mice treated with the antibiotics alone.

The same conclusion of Section I) may be applied in this case.

### III) Treatment with three doses of immunotherapeutic agent in liposome form simultaneously with isoniazid

Infected C57BL/6 type mice were divided into two groups:
- Treated with isoniazid alone at a dose of 25 mg /kg per day for 5 days per week for 8 weeks (Control), and
- Also treated with three 180 µg doses of the immunotherapeutic agent in liposome form, the object of the invention, administered intranasally

At week 9, the treatment with the antibiotic isoniazid begun and was continued until week 17.

The doses of the immunotherapeutic agent in liposome form were administered at weeks 13, 15 and 17.

The mice were sacrificed at weeks 15 and 28 and the bacillary concentrations in the left lung and the spleen were established.

The bacillary concentration was significantly lower in the lungs of vaccinated animals after administration of one or three doses (corresponding to weeks 15 and 28, respectively), compared to the Control group.

The results obtained for the lungs following one dose of the immunotherapeutic agent (week 15) and following 3 doses (week 28), expressed in log₁₀ UFC/mL, are shown in Table 3:

**Table 3**

| Mice group | Week 15 (1 dose) | Week 28 (3 dose) |
|---|---|---|
| Control | 2.34±0.24 | 3.86±0.41 |
| Intranasal | 1.59±0.61 | * 3.48±0.18* |
| * = statistically significant value compared to the Control group, p<0.05 | | |

It may be seen that the mice treated with only one dose of the immunotherapeutic agent in liposome form administered by intranasal route, simultaneously with a treatment with the antibiotic isoniazid, show a considerably lower number of bacilli in the lungs than the mice treated with the antibiotic alone.

The same conclusion of Section I) may be applied in this case.

As regards the spleen, the bacillary concentration was significantly lower in vaccinated animals following the administration of the 3 doses (corresponding to week 28), compared to the Control group.

The results obtained for the spleen, expressed in log₁₀ UFC/mL, are shown in Table 4:

**Table 4**

| Mice group | Week 15 | Week 28 |
|---|---|---|
| Control | 1,47±0,44 | 3,84±0,48 |
| Intranasal | 1,41±0,58 | 3,43±0,29* |
| * = statistically significant value compared to the Control group, p<0.05 | | |

It may be seen that the mice treated with three doses of the immunotherapeutic agent, object of the invention, administered intranasally simultaneously with treatment with the antibiotic isoniazid showed a considerably lower number of bacilli in the lungs than mice treated with the antibiotic alone.

The same conclusion of Section I) may be applied in this case.

### IV) Comparative trial to study the effect of the antibiotics, the immunotherapeutic agent in liposome form and their interactions

Several trials with DBA/2 mice have been conducted, following a 2² factorial design under the conditions shown in Table 5:

**Table 5**

| Trial | Antibiotic | Immunotherapeutic agent in liposome form |
|---|---|---|
| 1 | No | No |
| 2 | Yes | No |
| 3 | No | Yes |
| 4 | Yes | Yes |

In trial 1, the infected mice were maintained without any treatment.

In trial 2, the infected mice received the antibiotic isoniazid alone at a dose of 25 mg/kg per day for 5 days per week for 4 weeks and rifampicin at a dose of 10 mg/kg per day for 5 days per week for 4 weeks, starting at week 9 after infection.

In trial 3, the infected mice were treated with three 180 µg doses of immunotherapeutic agent in liposome form alone, administered subcutaneously at weeks 9, 11 and 15 after infection.

In trial 4, treatment with the antibiotic isoniazid was begun at week 9 and was administered for 4 weeks. At week 13, the treatment with rifampicin was initiated and was concluded at week 17. At weeks 17, 19 and 21, three doses of the immunotherapeutic agent in liposome form, object of the invention, were administered.

At week 22, all the animals were killed and the bacillary concentrations in the left lung were established. The results obtained for the lungs are expressed in log₁₀ UFC/mL and are shown in Table 6:

Table 6

| Trial | Antibiotic | Immunotherapeutic agent in liposome form | log₁₀ UFC/mL |
|---|---|---|---|
| 1 | No | No | 5.37±0.27 |
| 2 | Yes | No | 3.29±0.8* |
| 3 | No | Yes | 5.69±0.22 |
| 4 | Yes | Yes | 0.69±0** |
| *= statistically significant value compared to trials 1,3 and 4 for p<0.05; | | | |
| **= statistically significant value compared to trials 1,2 and 3 for p<0.05 | | | |

It may be seen that the combined treatment of the antibiotics isoniazid and rifampicin with the immunotherapeutic agent in liposome form, object of the invention, causes a considerably higher reduction in the number of bacilli compared with the reduction found with any of the other two factors (antibiotics and immunotherapeutic agent in liposome form) alone.

Taking into account that the number of established bacilli includes all bacilli, both those in an active phase and those in a non-active phase, the treatment with the immunotherapeutic agent in association with other drugs would allow to reduce the time of treatment with those drugs, since it considerably reduces the number of bacilli that may change with time into an active phase.

## Claims

1. A method to obtain an immunotherapeutic agent that contains cell wall fragments from a virulent *Mycobacterium tuberculosis*-complex (MTB-C) strain, comprising the following steps:
- culture the virulent MTB-C strain for a period of at least three weeks and then,
- homogenize the cell culture in the presence of a non-ionic surfactant selected from the group consisting of alkylphenol ethoxylates and ethoxylated sorbitan esters.

2. A method according to claim 1 **characterized in that** the culture period ranges from 3 to 4 weeks.

3. A method according to claims 1 or 2 **characterized in that** the non-ionic surfactant is selected from octylphenol ethoxylates.

4. A method according to claim 3 **characterized in that** the non-ionic surfactant is octylphenol ethoxylate with 7-8 mol of ethylene oxide.

5. A method according to claims 1 to 4 **characterized in that** homogenization carried out in a buffered medium with a neutral pH.

6. A method according to claims 1 to 5 **characterized in that** further comprising the steps:
- separate the non-fragmented cells and the solubilized compounds by centrifugation,
- treat chemically or physically the fraction with the cell wall fragments in order to inactivate any remaining virulent cells, and
- freeze-dry the resulting immunotherapeutic agent.

7. An immunotherapeutic agent that contains cell wall fragments from a virulent *Mycobacterium tuberculosis*-complex (MTB-C) strain obtainable by a method according to any of the claims 1 to 6.

8. A pharmaceutical composition that comprises the immunotherapeutic agent of claim 7.

9. A pharmaceutical composition according to claim 8
**characterized in that** it contains the immunotherapeutic agent in the form of liposomes.

10. The use of the immunotherapeutic agent of claim 7 to prepare a medicament for the combined treatment of tuberculosis in association with other drugs.

11. The use according to claim 10 **characterized in that** the drugs are isoniazid and/or rifampicin.

## Patentansprüche

1. Verfahren zur Herstellung eines Immuntherapeutikums enthaltend Zellwandfragmente aus einem virulenten Stamm des *Mycobacterium tuberculosis*-Komplexes (MTB-C), das folgende Schritte umfasst:
- Kultivierung des virulenten Stamms von MTB-C über einen Zeitraum von mindestens drei Wochen und anschließend
- Homogenisierung der Zellkultur in Gegenwart eines nichtionogenen Tensids ausgewählt aus der Gruppe bestehend aus Alkylphenolethoxylaten und ethoxylierten Sorbitanestern.

2. Verfahren nach Anspruch 1, **dadurch gekennzeichnet, dass** der Kultivierungszeitraum im Bereich von 3 bis 4 Wochen liegt.

3. Verfahren nach Anspruch 1 oder 2, **dadurch gekennzeichnet, dass** das nichtionogene Tensid aus Octylphenolethoxylaten ausgewählt ist.

4. Verfahren nach Anspruch 3, **dadurch gekennzeichnet, dass** das nichtionogene Tensid Octylphenolethoxylat mit 7-8 Mol Ethylenoxid ist.

5. Verfahren nach Ansprüchen 1 bis 4, **dadurch gekennzeichnet, dass** die Homogenisierung in einem Puffermedium mit neutralem pH-Wert erfolgt.

6. Verfahren nach Ansprüchen 1 bis 5, **dadurch gekennzeichnet, dass** es weiterhin folgende Schritte umfasst:
- Abtrennung der nicht gespalteten Zellen von den löslich gemachten Verbindungen mittels Zentrifugation,
- chemische oder physikalische Behandlung der Fraktion mit den Zellwandfragmenten zur Deaktivierung gegebenenfalls noch vorhandener virulenter Zellen, sowie
- Gefriertrocknung des entstandenen Immuntherapeutikums.

7. Immuntherapeutikum mit Zellwandfragmenten aus einem virulenten Stamm des *Mycobacterium* tuberculosis-Komplexes (MTB-C), erhältlich nach einem Verfahren nach einem der Ansprüche 1 bis 6.

8. Pharmazeutische Zusammensetzung, enthaltend das Immuntherapeutikum aus Anspruch 7.

9. Pharmazeutische Zusammensetzung nach Anspruch 8, **dadurch gekennzeichnet, dass** sie das Immuntherapeutikum in Form von Liposomen enthält.

10. Verwendung des Immuntherapeutikums aus Anspruch 7 zur Herstellung eines Arzneimittels zur Kombinationsbehandlung von Tuberkulose in Verbindung mit anderen Arzneimitteln.

11. Verwendung nach Anspruch 10, **dadurch gekennzeichnet, dass** es sich bei den Arzneimitteln um Isoniazid und/oder Rifampicin handelt.

## Revendications

1. Une méthode pour obtenir un agent immunothérapeutique contenant des fragments de paroi de cellule d'une souche complexe virulente *Mycobacterium tuberculosis* (MTB-C) comportant les étapes suivantes:
- culture d'une souche virulente MTB-C durant une période d'au moins trois semaines et ensuite,
- homogénéiser la culture de cellule en présence d'un tensioactif non ionique choisi d'un groupe consistant en phénolethoxylates alkyle et d'esters de sorbitan ethoxylé.

2. Un méthode conformément à la revendication 1, **caractérisée en ce que** la période de culture s'étend sur 3 à 4 semaines.

3. Une méthode conformément la revendication 1 ou 2 **caractérisée en ce que** le tensioactif non ionique est choisi parmi des éthoxylates d'octylphénol.

4. Une méthode conformément la revendication 3 **caractérisée en ce que** le tensio-actif non ionique est de l'éthoxylate octylphénol avec 7-8 mols d'oxyde d'éthylène.

5. Une méthode conformément aux revendications 1 à 4 **caractérisée en ce que** l'homogénéisation intervient dans un moyen tampon ayant un pH neutre.

6. Une méthode conformément aux revendications 1 à 5, **caractérisée en ce qu'**elle comprend en plus les étapes:
- séparer les cellules non fragmentées et les composés solubilisés par centrifugation.
- traiter chimiquement ou physiquement la fraction avec les fragments de paroi de cellule pour inactiver toutes cellules virulentes restantes, et
- lyophiliser l'agent immunothérapeutique résultant.

7. Un agent immunothérapeutique contenant des fragments de paroi de cellule d'un complexe de souche virulente *Mycobacterium tuberculosis* (MTB-C) pouvant être obtenu par une méthode conformément à une quelconque des revendications 1 à 6.

8. Un composé pharmaceutique comportant l'agent immunothérapeutique de la revendication 7.

9. Un composé pharmaceutique conformément à la revendication 8 **caractérisé en ce qu'**il contient l'agent immunothérapeutique sous forme de liposomes.

10. L'usage de l'agent immunothérapeutique de la revendication 7 pour préparer un médicament pour le traitement combiné de la tuberculose associé à d'autres médicaments.

11. L'usage conformément à la revendication 10 **caractérisé en ce que** les médicaments sont l'isoniazide et /ou la rifampicine.
